# EUROPÄISCHE PATENTANMELDUNG

(11) **EP 1 419 788 A1**
(43) Veröffentlichungstag der Anmeldung: **19.05.2004**
(21) Anmeldenummer: 02025601.2
(22) Anmeldetag: 18.11.2002
(51) Int. Cl.: A61K 49/00, A61K 51/08, A61K 51/12

(54) **Kontrastmittel für die Lymphknoten-Darstellung**

(71) Anmelder: Till, Uwe Prof. Dr., 99087 Erfurt (DE)
(72) Erfinder: Till, Uwe Prof. Dr., 99087 Erfurt (DE)
(74) Vertreter: HOFFMANN - EITLE

(57) **Zusammenfassung**

Die Erfindung betrifft eine Zusammensetzung enthaltend Albuminpartikel, die zu 90 % oder mehr eine Teilchengröße im Bereich von 5 bis 1500 nm aufweisen und mit einem Bisazofarbstoff mit einer maximalen Absorptionswellenlänge im Bereich von 550 bis 680 nm, vorzugsweise Evans blue, angefärbt sind. Die Zusammensetzung wird als Kontrastmittel für die Lymphknoten-Darstellung, insbesondere von Sentinel-Lymphknoten, verwendet.

## Beschreibung

Die Erfindung betrifft eine Zusammensetzung, die angefärbte Albuminpartikel enthält, sowie ein Verfahren zu ihrer Herstellung, eine pharmazeutische Formulierung, umfassend diese Albuminpartikel-Zusammensetzung und die Verwendung der Zusammensetzung zur Herstellung eines Kontrastmittels für die Lymphknoten-Darstellung, insbesondere von Sentinel-Lymphknoten.

Die häufigsten bösartigen Tumoren des Menschen setzen ihre Tochtergeschwülste primär in dem zugehörigen Lymphabflussgebiet ab. Auf diese Weise entstehen Lymphknotenmetastasen. Bei der operativen Tumorentfernung werden daher in der Regel die Tumor-benachbarten Lymphknotenstationen so komplett wie möglich entfernt. Nachfolgend werden die entfernten Lymphknoten in der Pathologie auf Metastasen untersucht. Von dem Ergebnis hängt die weitere Behandlung ab. Neben dem operativen Aufwand und der großen Zahl histomorphologischer Untersuchungen kann dieses Verfahren vor allem nach der Operation mit einer erheblichen gesundheitlichen Beeinträchtigung der Patienten verbunden sein, wie etwa durch Behinderung des Lymphabstroms, Narbenbildung, Nervenschädigung usw.

Nach dem sogenannten Sentinel(=Wächter)-Lymphknoten-Konzept wird bei der Bildung von Tochtergeschwülsten primär immer der Lymphknoten befallen, der als erster im Abstrombereich des Tumors stationiert ist (Büchels HK, Vogt H, Wagner T, Steinfeld D, Sagassser J, Sentinel-Lymphonodektomie beim Mammakarzinom, Der Nuklearmediziner 1999, 22; 261-267). Bei Beschränkung auf die Entfernung dieses Knotens (oder dieser Knoten bei mehreren Abstromgebieten) und den während der Operation durch sogenannten Schnellschnitt geführten Nachweis einer Metastasenfreiheit dieses Knotens könnte die Operation in diesem Stadium beendet werden. Der operative und histomorphologische Aufwand und die Beeinträchtigung der Patienten wären somit weitaus geringer. Dieses "Sentinel-Lymphknotenprinzip" ist derzeit für die operative Entfernung von Melanomen weitgehend etabliert und befindet sich für viele andere Turmorarten in klinischer Erprobung (Vogt H, Wengenmair H, Kopp J, Dorn R, Gröber S, Heidenreich P, Der Sentinel-Lymphknoten (SLN): prä- und intraoperative nuklearmedizinische Diagnostik, Der Nuklearmediziner 1999, 22, 233-242; Balch CM, Lange JR, Lymphatic Mapping and Sentinel Node Lymphadenectomy for Cancer: An Overview, Annals of Surgical Oncology 2001, 8(9S), 1-4; Zervos EE, Burak WE, Lymphatic Mapping in Solid Neoplasms: State of the Art; Cancer Control 2002; (8)3, 189-202).

Zur Erfassung von Sentinel-Lymphknoten werden derzeit vor allem zwei methodische Prinzipien eingesetzt:

Zum einen werden in Lösung befindliche kolloidale Partikel durch Technetium-99m radioaktiv markiert und vor der Operation in die unmittelbare Umgebung des Tumors injiziert. Die Partikel werden dann über die Lymphwege abtransportiert und in dem bzw. den Sentinel-Lymphknoten gespeichert. Ihr Nachweis erfolgt meist in zwei Schritten: a) am Vortag der Operation durch Nachweis der Radioaktivität mittels Ganzkörperszintigrafie und b) während der Operation mit eigens dafür konstruierten Gamma-Sonden als Radioaktivitätsdetektoren. Die Nachteile dieser Methode sind allerdings beträchtlich (siehe Heidenreich P, Vogt H, Bachter D, Büchels H, Steinfeld D, Wawroschek F, Wengenmair H, Wagner T, Das Konzept des Wächterlymphknotens, Dt. Ärzteblatt 2001, 98; A534-540):
- Sie ist an das Vorhandensein einer nuklearmedizinischen Abteilung gebunden.
- Sie ist technisch außerordentlich aufwändig. Neben der Ganzkörperszintigrafie sind speziell konstruierte Gamma-Sonden notwendig, die je nach Operationsgebiet verschieden sein müssen.
- Sie ist mit einer Strahlenexposition von Operationsteam, Patienten und Pathologen verbunden und erfordert entsprechende Vorkehrungen.
- Die eindeutige Lokalisation des Sentinel-Lymphknotens während der Operation ist mitunter nicht möglich, wegen zu enger Verhältnisse oder wenn der Sentinel-Lymphknoten sehr nahe dem Injektionsort liegt, an dem die meiste Radioaktivität liegen bleibt und so den Lymphknoten überstrahlt.

Trotz dieser Nachteile wird - in Ermangelung besserer Alternativen - diese Methode gegenwärtig in vielen Krankenhäusern angewendet.

Zum anderen werden für die Lymphknotendarstellung biokompatible, blaue Farbstoffe in die unmittelbare Umgebung des Tumors kurz vor der Operation injiziert. Dadurch färben sich Lymphbahnen und -knoten. Aber auch diese Methode hat ihre Nachteile (Bachter D, Starz H, Volkmar C, Vogt H, Büchels H, Balda BR, Die Sentinel-Lymphonodektomie beim malignen Melanom, Der Nuklearmediziner 1999, 22; 245-252):
- Die gegenwärtig eingesetzten Farbstoffe färben zwar zunächst die Sentinel-Lymphknoten, werden dort aber nicht komplett zurückgehalten, sondern passieren sie. Sie färben daher später auch andere, entfernter liegende Lymphknoten, so dass der Sentinel-Lymphknoten nicht immer eindeutig festgestellt werden kann. Dadurch ist der Operateur unter Umständen gezwungen, unnötig viele Lymphknoten zu entfernen, was mit einer entsprechenden post-operativen Belastung für den Patienten verbunden ist.
- Die Farbstoffe gehen in das Blut über und werden über Galle und Urin ausgeschieden. Es kommt zur Blaufärbung der Patienten, die einige Tage anhalten kann.

Die Nachteile der beiden Verfahrensweisen können teilweise durch die Kombination beider Techniken vermindert werden, indem z.B. eine grobe Orientierung durch die Gamma-Sonde durch die Färbung der Lymphknoten verbessert wird (Zervos EE, Burak WE, Lymphatic Mapping in Solid Neoplasms: State of the Art; Cancer Control 2002; (8)3, 189-202). Mit einer solchen Kombination wurden bislang die besten Ergebnisse erreicht (Allan R, Sentinel node localization: do or dye alone? Br J Radiol 2001, 74; 475-477). Der notwendige apparative und präparative Aufwand ist aber dann sehr hoch.

Ausgehend von diesem Stand der Technik ist es das der Erfindung zugrundeliegende Ziel, eine Zusammensetzung für ein Verfahren der Lymphknotendarstellung bereitzustellen, das nicht die Nachteile der oben diskutierten, bekannten Verfahren aufweist. Insbesondere soll ein ebenso zuverlässiges und weniger aufwändiges Verfahren bereitgestellt werden als das bisher meistens eingesetzte radiologische Sentinel-Lymphknoten-Diagnostizierverfahren. Ferner soll erfindungsgemäß eine Zusammensetzung bereitgestellt werden, die sich für ein solches Verfahren einsetzen lässt.

Dieses Ziel wird erfindungsgemäß erreicht durch Bereitstellung einer Zusammensetzung enthaltend Albuminpartikel, die zu 90 % oder mehr eine Teilchengröße im Bereich von 5 bis 1500 nm aufweisen und mit einem Bisazofarbstoff angefärbt sind, der im Bereich des sichtbaren Lichts ein Wellenlängenabsorptionsmaximum im Bereich von 550 bis 680 nm hat. Ferner wird erfindungsgemäß ein Verfahren zur Herstellung solcher Partikel sowie eine pharmazeutische Formulierung bereitgestellt, die eine solche Zusammensetzung mit angefärbten Albuminpartikeln in steriler, vorzugsweise pyrogenfreier (insbesondere bei Volumina über 15 ml) und pharmazeutisch akzeptabler (unbedenklicher) Form enthält. Nach einem weiteren Aspekt der Erfindung wird ein Verfahren zur Entfernung von Sentinel-Lymphknoten zur Verfügung gestellt, bei dem man einem Patienten vor der Tumoroperation die erwähnte Zusammensetzung in die unmittelbare Umgebung des Tumors injiziert und während der Operation den angefärbten Sentinel-Lymphknoten diagnostiziert und herausoperiert.

Die Albuminpartikel weisen zu 90 % oder mehr, vorzugsweise zu über 95 % eine Teilchengröße im Bereich von 5 bis 1500 nm, vorzugsweise 25 bis 1000 nm, besonders bevorzugt 300 bis 600 nm, auf. Die Teilchengröße kann lichtmikroskopisch bestimmt werden. Dazu wird ein Tropfen der Suspension nativ auf einen Objektträger verbracht, mit einem Deckglas abgedeckt und im Phasenkontrastverfahren bei 1.000facher Vergrößerung beurteilt (z.B. mit Objektiv 100x und Okular 10x unter Verwendung von Immersionsöl). Zur Ermittlung der Partikelgröße mittels Skalierung ist eine entsprechende Software einzusetzen (z.B. Analysis® der Software Imaging System GmbH). Es sollten mindestens 500 Teilchen vermessen werden.

Die Methode erlaubt darüber hinaus eine Beurteilung der Partikelform, die sphärisch sein sollte. Bei sphärischen Teilchen entspricht die Teilchengröße dem Durchmesser; bei nicht-sphärischen Teilchen entspricht die Teilchengröße der Mittelung aus der längsten Seite (L) und der kürzesten Seite (K), d.h. (L+K)/2. Außerdem erlaubt die Methode, unerwünschte Artefakte wie z.B. Agglomerate zu erkennen. Eventuell vorhandene Agglomerate werden bei der Teilchengrößenbestimmung nicht berücksichtigt.

Um farbstofffreie Teilchen und eventuell vorhandene Verunreinigungen nicht irrtümlich mitzuerfassen, werden unter Anwendung der Fluoreszenzmikroskopie unter gleichen Bedingungen wie zuvor farbstoffhaltige Partikel ausgewählt.

Bei einer Exzitationswellenlänge von 550 nm emittiert Albumin-gebundenes Evans Blue bei 610 nm. Bei Verwendung geeigneter Filter (z.B. Mirror Unit U-MWIG2 im Systemmikroskop BX 61 von Olympus Optical Co., Ltd.) erscheinen dann nur die farbstoffhaltigen Partikel rot.

Albuminpartikel mit der oben genannten Teilchengrößenverteilung können durch Denaturierung von Albumin, vorzugsweise humanem Serumalbumin, und anschließendem Einstellen der Teilchengröße durch z.B. Ultraschallbehandlung hergestellt werden. Die Denaturierung erfolgt vorzugsweise durch Erhitzen, vorzugsweise auf 75 °C bis 100 °C. Anschließend wird die erhaltene Mischung in der Regel auf Raumtemperatur (25 °C) abgekühlt, bevor die Teilchengröße durch z.B. Ultraschallbehandlung und anschließendem Filtrieren eingestellt wird. Um ein Agglomerieren der Albuminpartikel zu verhindern, kann der Zusammensetzung vorzugsweise ein nicht-ionisches Tensid wie Pluronic (z.B. Pluronic F-68) hinzugesetzt werden. In der erfindungsgemäßen Zusammensetzung liegen vorzugsweise weniger als 10%; besonders bevorzugt weniger als 2%, der insgesamt vorhandenen Partikel in Agglomeraten vor. Entsprechende Verfahren sind in US 4,024,233 und US 4,187,285 beschrieben. Albuminpartikel-Zusammensetzungen mit geeigneter Teichengröße sind auch im Handel erhältlich, z.B. Solco® Nanocoll oder Albu-Res (Hersteller: Amersham Sorin S.r.l., Vercelli, Italien).

Die Anfärbung des Albumins durch den Bisazofarbstoff kann vor, während oder nach der Denaturierung des Albumins erfolgen. Letzteres trifft bei Verwendung handelsüblicher Partikel zu. Hierzu werden Partikel in z.B. Phosphatpuffer (8,2 mM Na₂HPO₄, 1,8 mM KH₂PO₄, 137 mM NaCl, pH 7,2) in einer Konzentration entsprechend 0,4 bis 4 mg (vorzugsweise z.B. etwa 2,5 mg bei Albu-Res bzw. etwa 0,5 mg bei Solco Nanocoll) Albumin pro ml Lösung suspendiert. Der Lösung wird der Bisazofarbstoff zugefügt, entsprechend einer Konzentration von 1 bis 10 mM, vorzugsweise 3 bis 5 mM (z.B. 4 mM). Die Mischung wird vorzugsweise mindestens 15 min bei Raumtemperatur stehen gelassen. Mit Farbstoff markierte (angefärbte) Albuminpartikel werden dann von überschüssigem freien Farbstoff abgetrennt, z.B. durch:
a) Säulenchromatographie über Sephadex G-50 fine (Pharmacia) mit Phosphatpuffer der o.g. Zusammensetzung. Der separierte Partikel/Farbstoff-Komplex kann bei Bedarf schonend eingeengt werden, z.B. mittels Vakuum-Zentrifugalverdampfer.
b) Filtration durch Filter mit einer Porengröße, die weit unterhalb der Partikelgröße liegt, z.B. 25 nm. Bei Verwendung von Filterkammern (z.B. von Millipore) wird Phosphatpuffer der o.g. Zusammensetzung nachgesetzt. Die Prozedur ist auch geeignet, den Phosphatpuffer bei Bedarf durch beliebige andere Lösungen zu ersetzen.

Die Anfärbung der Albuminpartikel durch den Bisazofarbstoff kann durch chemische und/oder physikalische Wechselwirkungen vermittelt werden. Unter dem Gesichtspunkt der Stabilität wären kovalente chemische Bindungen zwischen Protein und Farbstoff bevorzugt. Aber auch physikalische Wechselwirkungen, z.B. Protein-Liganden-Wechselwirkungen, hydrophobe Wechselwirkungen, Wasserstoffbrücken u.ä. können für eine zufriedenstellende Anfärbung der Albuminpartikel völlig ausreichen, solange der Farbstoff-Albumin-Komplex auch in vivo hinreichend stabil bleibt. Ein praktisches Maß für ausreichende Stabilität bzw. Bindungsfestigkeit kann beispielsweise sein, dass in der für die Applikation vorgesehenen Flüssigkeit in 24 Stunden bei 37°C nicht mehr als maximal 5% des gebundenen Farbstoffs freigesetzt werden.

Die erfindungsgemäßen Zusammensetzungen können dann für die Lagerung konserviert werden, wie z.B. in US 4,187,285 und US 4,024,233 für die dort beschriebenen Albumin-Zusammensetzungen beschrieben. Z.B. können die Zusammensetzungen sterilisiert, lyophilisiert und unter Stickstoffatmosphäre verpackt werden, oder es kann die zur Applikation vorgesehene Zusammensetzung in physiologischer Lösung in Ampullen verbracht und autoklaviert oder pasteurisiert werden.

Geht man von nativem Albumin aus, wird dieses zunächst vorzugsweise in z.B. Phosphatpuffer gelöst und dann zur Denaturierung des Albumins erhitzt, vorzugsweise auf 75 bis 100°C. Nach Abkühlung wird der Bisazofarbstoff hinzugegeben und gerührt. Das gefärbte denaturierte Albumin wird dann abgetrennt, z.B. durch Zentrifugation. Nach Aufnahme des Niederschlags in Wasser wird die so erhaltene Suspension dann einer Ultraschallbehandlung unterworfen, um die Partikel der gewünschten Größe zu erhalten. Eine Ermittlung der geeigneten Dauer und Stärke der Behandlung kann durch Bestimmung der Ausbeute an Partikeln geeigneter Größe erfolgen. Dazu wird die weiter vorn beschriebene lichtmikroskopische Methode empfohlen.

Für die Zwecke dieser Erfindung geeignete Bisazofarbstoffe sind substituierte Aromaten mit zwei Azogruppen, die im sichtbaren Bereich ein Absorptionsmaximum im Bereich von Wellenlängen zwischen etwa 550 und 680 nm haben. Farbstoffe mit einer solchen Absorptionswellenlänge sind in der Regel blau oder grün und somit für die Anwendung bei der Lymphknotendarstellung besonders kontrastreich. Die Extinktion des Farbstoffs bei der maximalen Absorptionswellenlänge sollte vorzugsweise so hoch sein, dass man den Farbstoff mit bloßem Auge im Gewebe gut erkennen kann, wenn er in der zur Injektion üblichen Menge appliziert wird.

Bisazofarbstoffe eignen sich in besonderem Maße für die Anfärbung von Proteinen (siehe B.Stopa et al., Supramolecular ligands: Monomer structure and protein ligation capability; Biochimie (1998) 80, 963-968). Die Bisazofarbstoffe weisen vorzugsweise eine hohe Bindefähigkeit an Protein auf. Bezogen auf humanes Immunglobulin G als Standardprotein kann das molare Farbstoff-Protein-Verhältnis wie folgt bestimmt werden (siehe B. Stopa et al., Biochimie (1998) 80, 963-968): Eine Mischung aus Protein (Human-IgG) und Farbstoff (100-facher molarer Überschuss) werden 20 Minuten lang bei 63°C für die Agglutination erhitzt (Rollett A., Bacher W., Azo dyes IV, Monatsh. 73 (1940), 20-24). Das Farbstoff-Protein-Verhältnis wird dann spektrophotometrisch in Proben gemessen, die durch eine Bio-Gel P-6-Säule in 0,1 M Phosphatpuffer (pH 7,4; NaCl 0,15 M) zur Entfernung von überschüssigem Farbstoff filtriert wurden. Der Messung geht ein Trypsin-Verdauungsschritt zur Lösung des Farbstoffes vom Protein voraus. Besonders bevorzugt ist ein Farbstoff-Protein-Verhältnis von mindestens 20, besonders bevorzugt 20 bis 90.

Evans blue(4,4'- Bis(1-amino-8-hydroxy-2,4-disulfo-7-naphthylazo)-3,3'-bitolyl-Tetranatriumsalz) ist aufgrund seiner starken Bindung an Albumin sowie aufgrund seiner physiologischen Verwendbarkeit (siehe z.B. El-Sayed et al., Re-evaluation of Evans blue dye dilution method of plasma volume measurement, Clin.Lab.Haem. 1995, 17, 189-194) in besonderer Weise als Farbstoff für die erfindungsgemäßen Zusammensetzungen geeignet. Evans blue wurde bereits in ungebundener Form für die Lymphknotendarstellung verwendet (siehe J.-Y. Bobin et al., Tagging Sentinel Lymph Nodes: a Study of 100 Patients with Breast Cancer, Eur.J.Cancer, Ausgabe.35, Nr.4, 569-573, 1999). Die Verwendung von Evans blue für die Lymphknotendarstellung wurde jedoch später als nachteilig beschrieben, insbesondere gegenüber Patentblau, und daher hat sich in der Praxis letzteres durchgesetzt (siehe z.B. Bachter et al., Die Sentinel-Lymphonodektomie beim malignen Melanom, Der Nuklearmediziner, 22, V245-252 1999). Bisher wurden sowohl Patentblau als auch Evans blue allerdings immer in ungebundener Form direkt verwendet, während ihre Verwendung zum Anfärben von Partikeln und der Einsatz solcher Partikel für die Lymphknoten-Darstellung nicht beschrieben oder vorgeschlagen wurde.

Obwohl aus wirtschaftlichen und gesundheitlichen Gründen die Verwendung radiologischer Diagnoseverfahren weniger bevorzugt ist, kann die erfindungsgemäße Zusammensetzung neben den erfindungsgemäß angefärbten Albuminpartikeln zusätzlich auch Albuminpartikel, die radioaktiv markiert sind, beispielsweise mit Technetium-99 m, enthalten. Bei dieser Variante lässt sich insbesondere eine Zusammensetzung einsetzen, in der sowohl ^{99m}Technetium- als auch Bisazofarbstoff- markierte Albuminpartikel enthalten sind. Entsprechende Zusammensetzungen können unter Verwendung von Albuminpartikeln enthaltend Zinnionen (z.B. Zinn(II)-chlorid-2-hydrat; siehe US 4,024,233; US 4,187,285; Solco® Nanocoll; Albu-Res) durch Umsetzung mit einer Technetat [^{99m}Tc]-Lösung, z.B. Natriumpertechnetat [^{99m}Tc]-Lösung, erhalten werden. Aufgrund der kurzen Halbwertszeit von ^{99m}Technetium (6 Stunden) muss die Markierung dieser Partikel stets relativ kurz vor deren Anwendung erfolgen. ^{99m}Technetium- und Evans blue-markierte Partikel können daher getrennt hergestellt und vor der Injektion gemischt werden oder es wird der Partikel/Farbstoff-Komplex vor der Anwendung zusätzlich mit ^{99m}Technetium markiert.

Die Erfindung stellt auch eine pharmazeutische Formulierung bereit, die die erfindungsgemäße Albuminpartikel-Zusammensetzung in steriler und pyrogenfreier Form enthält. Die Formulierung sollte aus medizinischen und zulassungsrechtlichen Gründen insbesondere frei von Hepatitis B-Oberflächenantigenen (HbsAg), Antikörpern für das humane Immundefizienz-Virus (anti-HIV 1/2) sowie Antikörpern für das Hepatitis C-Virus (anti-HCV) sein. Die Formulierung kann in Form einer anwendungsfertigen physiologisch akzeptablen Lösung, die vorzugsweise isotonisch sein sollte, bereitgestellt werden, oder in lyophilisierter Form.

Die Verwendung von nicht radioaktiv-markierten Albuminpartikeln bzw. der entsprechenden erfindungsgemäßen pharmazeutischen Formulierung ist insbesondere dann vorteilhaft, wenn die Lymphabflussrichtung des Tumors schon bekannt ist. Das ist - abhängig von der Art und der Lokalisierung des Tumors - oft der Fall. Solche Albuminpartikel können unmittelbar vor der Operation injiziert werden, so dass der bzw. die Sentinel-Lymphknoten während der Operation an der Blaufärbung erkannt werden können. Der gesamte technische, finanzielle und personelle Aufwand der Radionuklidtechnik kann dann entfallen.

Wenn die Abflussrichtungen des Tumors nicht sicher bekannt sind, ist dagegen die Verwendung von erfindungsgemäßen Albuminpartikel-Zusammensetzungen, die zusätzlich radioaktiv markierte Albuminpartikel (z.B. mit Technetium-99 m) enthalten, vorteilhaft, weil sicherer. Über das radioaktive Isotop werden vor der Operation durch Ganzkörperszintigrafie die Abflussrichtungen ermittelt. Da der bzw. die Sentinel-Lymphknoten gleichzeitig durch die erfindungsgemäßen Partikel angefärbt ist bzw. sind, sind die Knoten intraoperativ leicht auffindbar. Es entfällt somit in dieser Variante die zweite Injektion freier Farbstoffe und der Einsatz von Gamma-Sonden, deren Konstruktion, Anpassung und rechnergestützte Auswertung zur Ausschaltung der Hintergrundstrahlung oder Steigerung der Auflösung außerordentlich aufwändig ist (siehe Heidenreich et al, Das Konzept des Wächterlymphknotens, Dt.Ärzteblatt 2001, 98, A534-540).

Die Erfindung betrifft auch die Verwendung der erfindungsgemäßen Zusammensetzung zur Herstellung eines Kontrastmittels für die Lymphknotendarstellung, insbesondere die Darstellung des bzw. der Sentinel-Lymphknoten. Dieses Kontrastmittel kommt vorzugsweise zum Einsatz während der operativen Entfernung des Tumors, insbesondere eines Melanoms, Mammakarzinoms, Zervixkarzinoms oder Prostatakarzinoms.

In einem ganz besonders bevorzugten Aspekt betrifft die vorliegende Erfindung ein Kontrastmittel für die Markierung von Sentinel-Lymphknoten im menschlichen Gewebe, enthaltend Albuminpartikel, die zu über 95% eine Größe im Bereich von 25-1000 nm haben und mit dem Farbstoff Evans Blue angefärbt sind, in steriler und pyrogenfreier Form in einem pharmazeutisch akzeptablen wässrigen Lösungsmittel. In bestimmten Ausführungsformen kann dieses Kontrastmittel zusätzlich auch Albuminpartikel enthalten, die mit radioaktivem 99m Tc markiert sind.

Ferner betrifft die Erfindung in einem weiteren Aspekt ein verbessertes Verfahren zur operativen Tumorbehandlung, bei dem einem Patienten vor der Operation eine erfindungsgemäße pharmazeutische Zusammensetzung mit angefärbten Albuminpartikeln als Kontrastmittel durch Injektion in den Tumor oder vorzugsweise in seine unmittelbare Umgebung verabreicht wird, dann eine ausreichende Zeit zugewartet wird, damit die angefärbten Partikel den oder die Sentinel-Lymphknoten erreichen. Abhängig von Art und Sitz des Tumors liegt dies zwischen 10 min und 2 h. Auch die Applikation am Vortag der Operation ist möglich, wenn z.B. gleichzeitig 99m Technetium-markierte Partikel gegeben werden, um mittels Scintigraphie die Lymphabflussrichtung zu ermitteln. Während der Operation wird der Tumor zusammen mit dem oder den angefärbten Sentinel-Lymphknoten entfernt. Je nach dem Zustand der Sentinel-Lymphknoten kommt dann die operative Entfernung weiterer Lymphknoten in Betracht. Wenn der Sentinel-Lymphknoten metastasenfrei ist, ist es nicht erforderlich, noch weitere Lymphknoten zu entfernen.

Die Erfindung wird durch die nachstehenden nichtbeschränkenden Beispiele erläutert.

### Beispiele

### Beispiel 1:

Herstellung einer erfindungsgemäßen Albuminpartikel-. Zusammensetzung - ausgehend von Albu-Res (Nycomed Amersham Sorin)
1. 2,5 mg Albumin als Mikrokolloid aus einer Flasche Albu-Res werden in 1 ml Phosphatpuffer (8,2 mM Na₂HPO₄, 1,8 mM KH₂PO₄, 137 mM NaCl, pH 7,2) suspendiert.
2. 0,5 ml dieser Suspension werden mit 2 mg Evans blue (Merck) versetzt, gemischt und 15 min bei Raumtemperatur stehen gelassen.
3. Danach wird die Suspension auf eine Säule (d = 1 cm, h = 2,5 cm) aufgetragen, die mit Sephadex G-50 fine (Pharmacia) gefüllt ist, das mit dem Phosphatpuffer der o.g. Zusammensetzung äquilibriert ist.
4. Elution mit dem gleichen Phosphatpuffer und Fraktionierung zu je 1 ml. Der Evans blue/Albu-Res-Komplex erscheint im Außenvolumen.
5. Die Gipfelfraktionen (Nr. 2 bis 5) werden vereint und mittels Vakuum-Zentrifugalverdampfer auf 0,4 bis 0,5 ml konzentriert.

### Beispiel 2:

Herstellung einer erfindungsgemäßen Albuminpartikel-Zusammensetzung - ausgehend von humanem Serumalbumin
1. 2 ml eines 0,5 M Phosphatpuffers vom pH 5,4 werden mit bidestilliertem Wasser auf 100 ml aufgefüllt und 0,4 g humanes Serumalbumin zugesetzt (z.B. 2 ml Humanalbumin Kabi 20 %ig).
2. Die Lösung wird im Wasserbad unter ständigem Rühren für 20 min auf 80°C erhitzt und danach auf Raumtemperatur zurückgekühlt.
3. Zugabe von 0,2 g Evans blue (Merck) und 15 min bei Raumtemperatur unter leichtem Rühren stehenlassen.
4. Zentrifugation der Mischung und Verwerfen des Überstandes. Auffüllen mit bidestilliertem Wasser auf etwa das ursprüngliche Volumen und Resuspendieren des Niederschlages. Dann erneute Zentrifugation und Wiederholung dieser Prozedur bis im Überstand keine nennenswerte Blaufärbung mehr auftritt.
5. Aufnahme der präzipitierten Aggregate in ca. 100 ml bidestilliertem Wasser und Behandlung mit Ultraschall. Dauer und Stärke der Ultraschallbehandlung sind von vielen Faktoren abhängig und daher zu erproben. Kriterium ist die Ausbeute an Partikeln geeigneter Größe (s. Punkt 7). Typischerweise entstehen überwiegend sphärische Partikel von etwa 450 nm Durchmesser, mit einer Schwankungsbreite von 300 bis 600 nm.
6. Zur Abtrennung von Resten freien Farbstoffs und eventuell vorhandener zu kleiner Partikel erfolgt eine Filtration der Partikelsuspension durch Filter mit einer Porengröße von 25 nm unter Nachfüllen mit physiologischer Kochsalzlösung, so daß ein Endvolumen von ca. 25 ml eingestellt wird.
7. Ermittlung der Partikelgrößenverteilung im Rückstand über dem Filter durch Untersuchung einer Probe im Phasenkontrast- und/oder Fluoreszenzmikroskop mit Skalierung bei 1.000facher Vergrößerung, wie auf Seite 5 beschrieben.

### Beispiel 3:

Tierexperimentelle Darstellung von Sentinel-Lymphknoten

Versuchstiere sind weibliche Wistar-Ratten (Alter: 3 Monate, Gewicht 220 - 270 g).
1. Die Narkose wird mit Ether eingeleitet. Dann erhält das Tier Ketamin+Rompun (Mischungsverhältnis 1+1; 1 µl/g Körpergewicht) intramuskulär injiziert. Ketamin 100 mg/ml (Atarost); Rompun: 2 % (Bayer Vital)
2. In die rechte Vorder- und Hinterpfote der Tiere werden je 0,1 ml der gemäß Beispiel 1 hergestellten Zusammensetzung, der das zur Sentinel-Lymphknoten-Darstellung in klinischem Einsatz befindliche ^{99m}Technetium-markierte Albu-Res in einer Dosis von 1 MBq beigemischt ist, subkutan in den Ballen injiziert. Gleichzeitig werden in die linke Vorder- und Hinterpfote der Tiere je 0,1 ml der letztgenannten Zusammensetzung, ebenfalls in einer Dosis von 1 MBq, injiziert.
3. Nach einer Verteilungszeit von 60 min wird das Tier mit einer Überdosis an Ether getötet.
4. Die Lymphknoten beider Seiten werden frei präpariert, entfernt und entsprechend der Nummerierung in der Abbildung FIG.J-1 in: Hebel H, Stromberg MW [eds.] Anatomy and Embryology of the Laboratory Rat, BioMed Verlag Wörthsee, 1986, mit 1 bis 19b beziffert und geordnet.
5. Die Blaufärbung der Lymphknoten wird mit dem bloßen Auge durch den Beobachter bewertet (0: keine Färbung; 1: schwache Färbung; 2: starke Färbung) und die aufgenommene Radioaktivitätsmenge in einem Gamma-Counter gemessen.

Das Ergebnis eines repräsentativen Versuches ist in den Tabellen 1 und 2 dokumentiert, bezüglich der Radioaktivität (RA) einmal als Absolutwerte (Tabelle 1) und zum anderen in Prozent der gesamten gemessenen Radioaktivität (Tabelle 2). Daraus ergibt sich, dass sich Radioaktivität und Blaufärbung nach gleichem Muster verteilen und daher mit dem Partikel/Farbstoff-Komplex die Sentinel-Lymphknoten genau so sicher erfasst werden wie mit dem radiologischen Verfahren. Dies trifft ausnahmslos auf alle bisher dazu durchgeführten Tierversuche zu.

**Tabelle 1**

| **Versuche mit** ^{**99m**}**Tc- und Evans-Blue-markiertem Albures rechts: Evansblue/Albures +** ^{**99m**}**Tc/Albures; links: nur** ^{**99m**}**Tc/Albures** | | | |
|---|---|---|---|
| **Tierseite** | **rechts** | | **links** |
| **LK** | **RA (cpm)** | **Färbung** | **RA (cpm)** |
| 1 | 68,2 | 0 | 127,4 |
| 2 | 23,9 | 0 | 31,0 |
| 3 | 8,7 | 0 | 20,5 |
| 4 | - | - | - |
| **5a** | **1.132.749,4** | **2** | **420.716,7** |
| 5b | 97.785,7 | 1 | 108.669,6 |
| 5c | 48.775,6 | 1 | 291.815,6 |
| 6a | 405.990,1 | 2 | 270.372,0 |
| 6b | 74.718,8 | 0 | 1.116,1 |
| 6c | 5.325,6 | 0 | 1.290,7 |
| 7 | 10,0 | 0 | 19,4 |
| 8 | - | - | - |
| 9 | 55,7 | 0 | 58,3 |
| 10 | 38,8 | 0 | 29,1 |
| 11 | - | - | - |
| 12 | 157,6 | 0 | 48,2 |
| 13* | 335,5 | 0 | 335,5 |
| 14 | 11.185,3 | 0 | 11.967,2 |
| 15 | - | - | - |
| 16* | 3.190,4 | 0 | 3.190,4 |
| 17 | - | - | - |
| 18 | 168.591,6 | 1 | 76.596,4 |
| 19a | 256,7 | 0 | 387,7 |
| 19b | 332,6 | 0 | 363,8 |
| **Summe (cpm)** | **1.949.600,2** | | **1.187.155,6** |
| LK=Lymphknoten; RA=Radioaktivität; cpm=counts per minute LK 13 und 16 liegen medial und sind singulär (RA auf beiden Seiten eingetragen) | | | |

**Tabelle 2**

| Versuche mit ^{99m}Tc- und Evans-Blue-markiertem Albures | | | |
|---|---|---|---|
| rechts: Evansblue/Albures + ^{99m}Tc/Albures; | | | |
| links: nur ^{99m}Tc/Albures | | | |
| **Tierseite** | **rechts** | | **links** |
| **LK** | **RA (%)** | **Färbung** | **RA (%)** |
| 1 | 0,003% | 0 | 0,011% |
| 2 | 0,001% | 0 | 0,003% |
| 3 | 0,000% | 0 | 0,002% |
| 4 | - | - | - |
| **5a** | **58,102%** | **2** | **35,439%** |
| 5b | 5,016% | 1 | 9,154% |
| 5c | 2,502% | 1 | 24,581% |
| 6a | 20,824% | 2 | 22,775% |
| 6b | 3,833% | 0 | 0,094% |
| 6c | 0,273% | 0 | 0,109% |
| 7 | 0,001% | 0 | 0,002% |
| 8 | - | - | - |
| 9 | 0,003% | 0 | 0,005% |
| 10 | 0,002% | 0 | 0,002% |
| 11 | - | - | - |
| 12 | 0,008% | 0 | 0,004% |
| 13* | 0,017% | 0 | 0,028% |
| 14 | 0,574% | 0 | 1,008% |
| 15 | - | - | - |
| 16* | 0,164% | 0 | 0,269% |
| 17 | - | - | - |
| 18 | 8,647% | 1 | 6,452% |
| 19a | 0,013% | 0 | 0,033% |
| 19b | 0,017% | 0 | 0,031% |
| **Summe (cpm)** | **100,000%** | | **100,000%** |

## Patentansprüche

1. Zusammensetzung, enthaltend Albuminpartikel, die zu 90 % oder mehr eine Teilchengröße im Bereich von 5 bis 1500 nm aufweisen, **dadurch gekennzeichnet, dass** die Albuminpartikel mit einem Bisazofarbstoff angefärbt sind, der im sichtbaren Licht ein Absorptionswellenlängen-Maximum im Bereich von 550 bis 680 nm hat.

2. Zusammensetzung nach Anspruch 1, **dadurch gekennzeichnet, dass** der Bisazofarbstoff Evans Blue ist.

3. Zusammensetzung nach einem der vorhergehenden Ansprüche, **dadurch gekennzeichnet, dass** die Albuminpartikel zu 90 % oder mehr eine Teilchengröße im Bereich von 25 bis 1000 nm aufweisen.

4. Zusammensetzung nach einem der vorhergehenden Ansprüche, **dadurch gekennzeichnet, dass** die Zusammensetzung zusätzlich Albuminpartikel enthält, die radioaktiv markiert sind.

5. Zusammensetzung nach Anspruch 4, **dadurch gekennzeichnet, dass** die Albuminpartikel sowohl mit Bisazofarbstoff angefärbt als auch radioaktiv markiert sind.

6. Verfahren zur Herstellung einer Zusammensetzung nach einem der vorhergehenden Ansprüche, umfassend die Schritte:
- Vermischen des Bisazofarbstoffes mit Albuminpartikeln, die zu 90 % oder mehr eine Teilchengröße im Bereich von 5 bis 1500 nm aufweisen,
- Rühren der erhaltenen Mischung,
- Abtrennen des nicht-gebundenen Farbstoffes durch Säulenchromatographie oder Filtration; und
- Einengen der erhaltenen Zusammensetzung.

7. Verfahren zur Herstellung einer Zusammensetzung nach einem der Ansprüche 1 bis 5, umfassend die Schritte:
- Erhitzen einer wässrigen Albumin-Lösung auf 75 bis 100°C;
- Vermischen des Bisazofarbstoffes mit dieser Lösung;
- Abtrennen und Suspendieren des so erhaltenen gefärbten Albumins in Wasser;
- Ultraschallbehandeln der so erhaltenen Suspension zur Herstellung von Partikeln der gewünschten Größe von 5 bis 1500 nm, vorzugsweise 25 bis 1000 nm, besonders bevorzugt 300 bis 600 nm;
- Entfernung eventuell vorhandener zu kleiner Partikel oder von freiem Farbstoff durch Filtration;
- Lichtmikroskopische Kontrolle der Partikelgrößenverteilung.

8. Pharmazeutische Zusammensetzung, umfassend die Zusammensetzung nach einem der Ansprüche 1 bis 5 in steriler, pyrogenfreier Form in einem pharmazeutisch akzeptablen Lösungsmittel.

9. Verwendung der Zusammensetzung nach einem der Ansprüche 1 bis 5 zur Herstellung eines Kontrastmittels für die Lymphknoten-Darstellung.

10. Verwendung nach Anspruch 9, wobei das Kontrastmittel für die Darstellung von Sentinel-Lymphknoten dient.

11. Verwendung nach einem der Ansprüche 9 oder 10, wobei die Lymphknoten-Darstellung bei Patienten mit Tumoren erfolgt, die in Lymphknoten metastasieren, z.B. Melanome, Mamma-, Zervix- oder Prostatakarzinome.

12. Kontrastmittel für die Markierung von Sentinel-Lymphknoten im menschlichen Gewebe, enthaltend Albuminpartikel, die zu über 95% eine Größe im Bereich von 25-1500 nm haben und mit dem Farbstoff Evans Blue angefärbt sind, in steriler und pyrogenfreier Form.

13. Kontrastmittel nach Anspruch 12, das zusätzlich Albuminpartikel enthält, die mit radioaktivem 99m Tc markiert sind.

14. Kontrastmittel nach Anspruch 12 oder 13 in lyophilisierter Form.

15. Kontrastmittel nach Anspruch 12 oder 13 als Partikelsuspension in einem pharmazeutisch akzeptablen wässrigen Lösungsmittel.

16. Verfahren zur operativen Tumorbehandlung, insbesondere von Mammakarzinomen, Melanomen, Zervix- und Prostatakarzinomen, umfassend die Schritte des Injizierens einer pharmazeutischen Zusammensetzung gemäß Anspruch 8 oder 12-15 vor der Operation in den Tumor oder die unmittelbare Tumorumgebung, Zuwarten während eines Zeitraums, der für das Abfließen der Zusammensetzung durch das Lymphsystem bis mindestens zum Sentinel-Lymphknoten ausreicht, und dann operative Entfernung des Tumors und des bzw. der angefärbten Sentinel-Lymphknoten und Überprüfung derselben auf Metastasen.
